# EUROPEAN PATENT SPECIFICATION

(11) **EP 1 364 032 B1**
(45) Date of publication and mention of the grant of the patent: **11.10.2006**
(21) Application number: 02719782.1
(22) Date of filing: 08.02.2002
(51) Int. Cl.: C12N 15/81, C12N 15/62, C12P 21/02, C12P 21/06, C07K 14/815, C07K 14/62, C12N 15/17, C12N 1/19

(54) **USE OF FUSION PROTEINS WHOSE N-TERMINAL PART IS A HIRUDIN DERIVATIVE FOR THE PRODUCTION OF RECOMBINANT PROTEINS VIA SECRETION BY YEASTS**
VERWENDUNG VON FUSIONSPROTEINEN, DEREN N-TERMINALER TEIL AUS EINEM HIRUDINDERIVAT BESTEHT, ZUR HERSTELLUNG REKOMBINANTER PROTEINE DURCH SEKRETION DURCH HEFEN
UTILISATION DE PROTEINES DE FUSION DONT LA PARTIE N-TERMINALE EST UN DERIVE DE HIRUDINE A DES FINS DE PRODUCTION DE PROTEINES RECOMBINANTES AU MOYEN DE LA SECRETION PAR DES LEVURES

(30) Priority: 20.02.2001 DE 10108211
(43) Date of publication of application: 26.11.2003
(73) Proprietor: Sanofi-Aventis Deutschland GmbH, 65929 Frankfurt am Main (DE)
(72) Inventor: HABERMANN, Paul, 65817 Eppstein (DE)
(86) International application number: PCT/EP2002/001308
(87) International publication number: WO 2002/070722

(56) References cited:
- EP-A- 0 158 564
- EP-A- 0 511 393
- WO-A-02/04486
- WO-A-91/09125
- WINTER J ET AL: "Increased production of human proinsulin in the periplasmic space of Escherichia coli by fusion to DsbA" JOURNAL OF BIOTECHNOLOGY, ELSEVIER SCIENCE PUBLISHERS, AMSTERDAM, NL, vol. 84, no. 2, 30 November 2000 (2000-11-30), pages 175-185, XP004220615 ISSN: 0168-1656

## Description

The development of optimized processes for producing pharmaceuticals on the basis of recombinant proteins is a task which has to do justice to the following points of view. Firstly, a process ought to be as cost-effective as possible and, secondly, the product ought to be of the highest purity. In this connection, the choice of expression system determines the course of the particular production process, and it is obvious to the skilled worker that the development of novel techniques in protein chemistry and the wide variety of biochemical possibilities and new combinations of known techniques always make improvements of existing processes possible. The expression of relevant proteins of this kind in yeasts is widely used here.

The production of proteins such as insulin, GM-CSF (Leukine ®) and hirudin (Refludan ®) is an example of the successful development of genetic engineering processes which are based on the synthesis of the particular protein or precursors thereof in yeast. Generally, yeasts can directly synthesize particularly hirudins with good yields which are on the gram scale when using Hansenula polymorpha (Weydemann et al. Appl. Microbiol Biotechnol. 44: 377 -385, 1995) or Pichia pastoris (Rosenfeld et al. Protein Expr. Purif :4 , 476 -82, 1996).

In European Patent Application 0 158 564 A1vectors for the expression of Hirudin or a hirudin analogue are disclosed.

International Patent Application WO 91/09125 discloses that inactive fusion proteins are activatable by enzymes of the clotting cascade to have fibrinolytic and/or clot formation inhibition activity. For example, a fusion protein comprising two Hirudin or streptokinase molecules, linked by a cleavable linkage sequence, may be cleaved to yield anti-thrombotic hirudin or fibrinolytic streptokinase by thrombin or Factor Xa.

Fibrinolytic or clot formation inhibition activity is therefore directed to the site of clot formation.

Winter et al. (Journal of Biotechnology 84 (2000) 175-185) describe a method for the production of high amounts of soluble, native human proinsulin in E. coli by fusing proinsulin to the C-terminus of the periplasmic disulfide oxidoreductase DsbA via a trypsin cleavage site.

Surprisingly, we have found now that fusion proteins containing hirudin or hirudin derivatives at the N terminus can be exported from yeasts with good yields similar to those of hirudin itself. Yields are based on molarity. This means that a host/vector system producing yields of 100 mg of native hirudin per liter can produce approx. 180 mg fusion protein per liter, which is made of hirudin and, for example, mini-proinsulin which is as described in EP-A 0 347 781. Surprisingly, hirudin is biologically active and mini-proinsulin is present in the correctly folded three-dimensional form. If the two proteins are fused via a linker of amino acids which are specifically recognized by endoproteases which efficiently cleave the fusion protein at no other position, then the protein of interest can be cleaved off directly and in active form. In the case of insulin production, the linker between hirudin and mini-proinsulin preferably contains arginine at the carboxy-terminal end. In simultaneous processing it is then possible by conversion with trypsin to cleave off the fusion part and convert proinsulin to mono-Arg insulin. The invention thus relates to a DNA-molecule (alternative term: expression cassette) of the form:

Pₓ - Sₓ - Bₙ - (ZR) - Hir (AsₘR)- protein (Y) - T,

- Pₓ: is any promoter DNA sequence, selected in such a way that optimal yields of the protein of interest become achievable;
- Sₓ: is any DNA encoding a signal sequence or leader sequence which allows optimal yields;
- Bₙ: is 1-15 amino acid codons or a chemical bond;
- Z: is the codon of an amino acid selected from the group comprising Lys and Arg;
- R: is an Arg codon or a chemical bond;
- Asₘ: is a chemical bond or m amino acid codons, where m = 1-10;
- Hir: is a DNA sequence coding for hirudin or a hirudin derivative which is at least 40 % homologous to natural hirudin, wherein the hirudin derivative can be exported from yeast with good yields similar to those of Hirudin itself;
- protein Y: is a DNA sequence encoding a mini-proinsulin and
- T: is an untranslated DNA sequence having a stabilizing effect on the mRNA.

Preferred proteins Y are polypeptides such as mini-proinsulin derivatives, interleukins or lymphokines or interferons. The expression cassette is preferably introduced into yeasts. Said expression cassette may have one or more copies stably integrated into the particular yeast genome or may be present extrachromosomally on a multicopy vector or on type of minichromosomal element.

Another embodiment of the invention is a fusion protein encoded by any of the above-mentioned DNA molecules.

A further embodiment of the invention is a multicopy vector and a plasmid comprising the above-mentioned DNA-molecule.

An additional embodiment of the invention is a host cell comprising the above-mentioned DNA-molecule, or the above-mentioned multicopy vector or the above-mentioned plasmid, as a part of its chromosome, as a part of a mini-chromosome, or extra-chromosomally, wherein preferrentially said host cell is a yeast, in particular selected from the group comprising of S. cerevisiae, K. lactis, H. polymorpha and P. pastoris.

Another embodiment of the invention is a process of fermenting the above-mentioned fusion protein, in which
(a) the above-mentioned DNA-molecule, the above-mentioned multicopy vector, or the above-mentioned plasmid is expressed in an above-mentioned host cell, and
(b) the expressed fusion protein is isolated from the supernatant of the cell culture,
wherein in particular after completion of fermentation, the pH is adjusted to 2,5-3,5 in order to precipitate non-desired proteins and the expressed fusion protein is isolated from the supernatant of the precipitation.

Another embodiment of the invention is the above mentioned process, in which process after separating the fermentation supernatant from the host cells, the host cells are repeatedly cultured in fresh medium, and the released fusion protein is isolated from each supernatant obtained during cultivation.

Another embodiment of the invention is the above mentioned process, wherein a process step for concentrating the expressed protein in the supernatant after precipitation is selected from a group comprising microfiltration, hydrophobic interaction chromatography and ion exchange chromatography.

An additional embodiment of the invention is a process for preparing insulin, in which
(a) the above-mentioned fusion protein is expressed and isolated according to the above-mentioned process;
(b) the fusion protein is treated with trypsin and carboxypeptidase B; and
(c) insulin is isolated from the reaction mixture of step (b).

The expression system described below serves as an example. It is obvious to the skilled worker that, in order to introduce the expression cassette into said selected system, the appropriate recombinant DNA constructions must be made depending on the type of host system selected. Accordingly, industrial fermentation can be optimized in relation to the selected host/vector system.

Leeches of the type Hirudo have developed, for example, various isoforms of the thrombin inhibitor hirudin. Hirudin has been optimized for pharmaceutical requirements by artificial variation of the molecule, for example exchange of the N-terminal amino acid (e.g. EP-A 0 324 712). The invention includes the use of hirudin and hirudin variants. Particular embodiments of the invention use one of the natural hirudin isoforms (the natural isoforms are together denoted "hirudin"). A natural isoform is, for example, Val-Val-hirudin or Ile-Thr-hirudin. Other embodiments of the invention use a variant of a natural hirudin isoform. A variant is derived from a natural hirudin isoform but contains, for example, additional amino acids and/or amino acid deletions and/or amino acid exchanges compared with the natural isoform. A hirudin variant may contain alternating peptide segments of natural hirudin isoforms and new amino acids. Hirudin variants are known and are described, for example, in DE 3 430 556. Hirudin variants are commercially available in the form of proteins (Calbiochem Biochemicals, Cat. no.377-853, -950-960).

Frequently, fusion proteins containing hirudin show surprisingly good solubility in acidic medium, and this leads to distinct advantages regarding the chemical workup of the protein. Firstly, the many components of the supernatant are precipitated under said conditions and, secondly, most peptidases or proteases are inactive. Thus, acidifying the fermentation broth at the end of the operation makes it possible to directly separate unwanted supernatant proteins together with the host cells from the fusion protein and, in a further step, to concentrate said fusion protein. This is likewise a subject of the invention.

At the end of the fermentation, the folding process may not yet be 100% complete. The addition of mercaptan or, for example, cysteine hydrochloride can complete the process. This is likewise a subject of the invention.

The examples below describe the invention in more detail, without being restrictive.

### Example 1: Construction of an expression cassette encoding a fusion protein made of Leu - hirudin (Refludan®) - Arg - mini-proinsulin

Starting materials are the plasmids pK152 (PCT/EP00/08537), pSW3 (EP-A 0 347 781) and the recombinant yeast plasmid derivative coding for bovine interleukin 2 (Price et al. Gene 55, 1987). The yeast plasmid is distinguished by carrying the α factor leader sequence under the control of the yeast ADH2 promoter. This sequence is followed by the bovine interleukin 2 cDNA sequence which is connected via a Kpnl restriction enzyme recognition site and which contains, after manipulation, an Ncol restriction enzyme recognition site in the untranslated 3' end which is unique in the vector. Thus, the cDNA sequence can readily be removed from the plasmid via Kpnl/Ncol cleavage. Since good expression yields have been reported, it can be assumed that the remaining 3' interleukin 2 sequence (as T) has a stabilizing effect on the mRNA and thus need not be replaced by a yeast specific terminator sequence. Plasmid pK152 carries the DNA sequence coding for Leu-hirudin (Refludan) kodiert and plasmid pSW3 carries the DNA sequence for mini-proinsulin. The gene sequence to be encoding hirudin - Lys Arg - mini-proinsulin is first prepared by means of PCR technology. For this purpose, 4 primers are prepared with the aid of the Expedite^{™} DNA synthesis system:
i. hir_insf1 (SEQ ID NO: 1, encoded protein segment: SEQ ID NO: 2)
ii. hir_insrev1 (SEQ ID NO: 3)
   5'- CCTCACAAGTG TTGGTTAACA AA TCG CT GAAGGTATTC CTCAGGGAT-3'
iii. hirf1 (SEQ ID NO: 4, encoded protein segment: SEQ ID NO: 5)
iv. insnco1 rev (SEQ ID NO: 6)
   5'- TTTTTTCCAT GGGTCGACTATCAG

Primer hir_insf1 describes the junction between codons for the terminal amino acids of hirudin (59 - 65) and the insulin sequence B1 - B7 via the Arg linker (codon in bold type). Primer hir_insrev1 is 100% complementary thereto. Primer hirf1 codes for the start of the hirudin gene extended to the Kpnl cleavage site as described in EP-A 0 324 712. Primer insncoirev marks the 3' end of the synthetic mini-proinsulin according to EP-A 0 347 781.

Two standard polymerase chain reactions are carried out using the primer pairs hirf1/hir_insrev1 with DNA of plasmid pK152 as template and hir_insf1 / insncoirev with DNA of plasmid pSW3 as template. The reactions are carried out in 100µl PCR buffer with, in each case, 200 nmol of primer, 1 µl of polymerase and 100ng of vector. Step 1 is a 2-minute incubation at 95°C.
This is then followed by 25 cycles of 30" at 95°C, 30" at 55°C and 30" at 72°C. The last cycle is followed by an incubation at 72 °C for 3 minutes, and the reaction is subsequently stopped. Since the primers hir_ insrevkr and hir_insfkr are 100% complementary, the DNA products of the two products overlap according to said sequence so that in a third reaction, using the products of the first two reactions as templates and the primers hirf1 and insncoirev, a DNA fragment is formed, which encodes hirudin and mini-proinsulin separated by Arg. The PCR fragment is digested by the enzymes Kpnl und Ncol and then, in a T4 ligase reaction, inserted into the pαADH2 vector opened by Kpn1 / Ncol. In analogy to example 7 of EP-A 0 347 781, competent E. coli MM294 cells are then transformed with the ligation mixture. Plasmid DNA is then isolated from two clones for characterization by means of DNA sequence analysis. After confirmation of the inserted DNA sequence, DNA of a plasmid preparation is used to transform cells of baker's yeast strain Y79, according to said example. However, when using the pαADH2 vector, introduction of the vector is followed by selecting for complementation of the trp1-1 mutation, in contrast to said example. For another control, plasmid DNA is reisolated from yeast transformants and analyzed by means of restriction analysis. The expression vector constructed is denoted pADH2Hir_Ins. Expression is carried out according to example 4. The fusion protein is found in the supernatant.

### Example 2: Construction of an expression cassette encoding a fusion protein made of Leu - hirudin (Refludan) - Gly Asn Ser Ala Arg - mini-proinsulin

The example demonstrates a way of modifying the trypsin recognition site between hirudin derivative and mini-proinsulin. The construction is carried out according to example 1.

Two new oligonucleotides are synthesized:
Hir_insf (SEQ ID NO: 7, encoded protein segment: SEQ ID NO: 8)
Hir_insrev (SEQ ID NO: 9)

Two polymerase chain reactions are carried out using the primer pairs hirf1/hir_insrev1 with DNA of plasmid pK152 as template and hir_insf1 / insncoirev with DNA of plasmid pSW3 as template. In a third reaction, using the products of the first two reactions as templates and the primers hirf1 and insncoirev, a DNA fragment is formed, which encodes hirudin and mini-proinsulin separated by the linker Gly Asn Ser Ala Arg. The product of PCR3 is subsequently cleaved by Kpnl and Ncol, introduced into the appropriately opened pαADH2 vector and characterized according to example 1. The plasmid is denoted pADHH_GNSA_Ins. Cells are transformed with the plasmid DNA. Expression is carried out according to example 3. The fusion protein is found in the supernatant.

### Example 3: Expression of the recombinant products in the baker's yeast system

The expression is devided into two phases. Firstly, a preculture is cultivated in yeast minimal medium. The medium has the following composition per liter:
6.7 g - yeast nitrogen base (without amino acids)
5.0 g - casamino acids (vitamin-free)
0.008% - adenine
0.008% - uracil
2% - glucose

The main or expression culture is inoculated with an aliquot of the preculture.

The main culture medium contains per liter:
10 g - yeast extract
20 g - peptone
0.008% - adenine
0.008% - uracil
4% - glucose

Using the media described, expression is carried out in a shaken flask in the following way: 0.3 ml of a preculture which has been cultivated overnight is diluted with 80 ml of prewarmed medium and incubated with vigorous shaking at 30°C for approx. 24 h. In each case, 1 ml of the culture produced in this way is then centrifuged, after determining the optical density, and, after removing the cells, the supernatant is lyophilized and analyzed by means of SDS -PAGE. The biologically active hirudin content is determined by carrying out a thrombin inhibition assay. An alternative fermentation protocol provides for the cells to be removed by filtration or careful centrifugation. While isolating the protein of interest from the medium, the cells are provided with fresh prewarmed main culture medium containing alcohol and not more than 0.5% of glucose as carbon sources, and thus fermentation is continued without interruption. This step can be repeated up to 5 times.

### Example 4: Cloning and expression of the hirudin - Arg - mini-proinsulin fusion protein in the P. pastoris system

Invitrogen® sells a cloning and expression kit for preparing recombinant proteins with the aid of the P. pastoris system. For this, a detailed technical protocol regarding preparation and subsequent expression of a P. pastoris system for the production of a desired recombinant protein is provided so that only the construction of the expression vector encoding the desired protein has to be described when following said protocols. The EasySelect^{™} Pichia expression kit (catalog no. K1740-01) is used.

The pPICZαA vector is part of the kit. Opening the vector by the restriction enzymes Xhol and Sacll makes it possible to append, similar to example 1, a protein of interest to the alpha factor leader sequence and to test for secretion into the supernatant. Cloning of the fusion protein requires two primers. Primer pichia_H_If1 (SEQ ID NO: 10) has the sequence: Primer pichia_H_Irev2 (SEQ ID NO: 11) has the sequence:

The template used is DNA of plasmid pADH2Hir_Ins. A standard PCR with both primers produces a DNA product which contains the sequence hirudin - Arg - mini-proinsulin extended by the XhoI and Sacll integration sites. If the DNA product is cleaved appropriately and the fragment is isolated, said fragment can be inserted into the opened vector DNA in a T4 DNA ligase reaction. In deviation from the manufacturer's protocol, E. coli strain MM294, described in example 1, is transformed with the ligation mixture and recombinant colonies are screened for successful transformation on zeocine selection plates. Plasmid DNA is reisolated from clones and then characterized by means of restriction and DNA sequence analysis. Using the plasmid constructed in this way, a P. pastoris expression clone for production of the fusion protein is then prepared, following the manufacturer's instructions.

### Example 5: Thrombin inhibition assay

The hirudin concentration is determined according to the method of Grießbach et al. (Thrombosis Research 37, pp. 347-350 , 1985 ). For this purpose, specific amounts of a Refludan standard are included in the measurements in order to establish a calibration curve from which the yield in mg/l can be determined directly. The biological activity is also a direct measure for correct folding of the proinsulin component of the fusion protein. Alternatively, it is possible to use a proteolytic S. aureus digestion and subsequent analysis in an RP-HPLC system to determine the correct S-S bridge formation.

### Example 6: Purification of the fusion protein

After completion of the fermentation, the pH is adjusted to 2.5 - 3. In contrast to most other polypeptides found in the supernatant due to either spontaneous lysis of host cells or secretion, the fusion protein is surprisingly not precipitated at pH 2.5-3. The culture medium is therefore acidified appropriately and then, after completion of the precipitation, the precipitate and the cells are removed by centrifugation or by microfiltration and concentrated. Subsequently, the medium is adjusted to pH 6.8 and the fusion protein content is determined in parallel by analytical HPLC measurement. The determination is followed by adding trypsin to the supernatant so that trypsin is at approx. 1 µg per 1-1.5 mg of fusion protein. After incubation at room temperature for approx. 4 hours, purification is carried out by cation exchange chromatography at pH 3.5 in the presence of 2-propanol. Elution is carried out in the buffer by applying a gradient of from 0.15 to 0.45 M. Mono-Arg-insulin is eluted at approx. 0.3 M. After 1:1 dilution, mono-Arg-insulin is precipitated from the insulin-containing fractions at approximately pH 6.8 with the addition of a 10% strength ZnCl₂ solution. Insulin is filtered off and then dissolved in 0.05 M Tris-HCl (pH 8.5) resulting in a 2 mg/ml solution. Then the amount of approximately 1 unit of carboxypeptidase B per 100ml solution is added and the reaction is carried out with gentle stirring. The pH is then adjusted to pH 5.5 with citric acid, and insulin is crystallized in the presence of ZnCl₂. The crystals are removed, dissolved and, after purification by RP-HPLC, insulin is purified again by crystallization.

### Example 7: Processing of the fusion protein directly in the culture medium

At the end of the expression period, the culture medium is adjusted to pH 6.8 and trypsin is then added with stirring so that a final concentration of 4-8 mg per liter is established. After incubation for approx. 4 hours, the fermentation broth treated in this way is adjusted to pH 2.5-3. After 1-6 hours of precipitation, the pH is raised to 3.5, and the mono-Arg-insulin formed is purified via cation exchange chromatography in the presence of 30% 2-propanol. Elution is carried out by means of an NaCl gradient of 0.05-0.5 M salt. The product-containing fractions are diluted 1:1 with H₂O and then ZnCl₂ is added, so that a 0.1 % strength ZnCl₂ solution is formed. Mono-Arg-insulin precipitates at approx. pH 6.8 and by way of example is converted to insulin according to example 6.

## Claims

1. A DNA molecule of the form :
Pₓ - Sₓ - Bₙ - (ZR) - Hir (AsₘR)- protein (Y) - T
where
Pₓ is any promoter DNA sequence, selected in such a way that optimal yields of protein of interest become achievable;
Sₓ is any DNA encoding a signal sequence or leader sequence which allows optimal yields;
Bₙ is 1-15 amino acid codons or a chemical bond;
Z is the codon of an amino acid selected from the group comprising Lys and Arg;
R is an Arg codon or a chemical bond;
Asₘ is a chemical bond or m amino acid codons, where m = 1-10;
Hir is a DNA sequence coding for hirudin or a hirudin derivative which is at least 40 % homologous to natural hirudin, wherein the hirudin derivative can be exported from yeast with good yields similar to those of Hirudin itself;
protein Y is a DNA sequence encoding a mini-proinsulin and
T is an untranslated DNA sequence having a stabilizing effect on the mRNA.

2. Fusion protein encoded by any of the DNA molecules according to claim 1.

3. Multicopy vector comprising the DNA-molecule of claim 1.

4. Plasmid comprising the DNA-molecule of claim 1.

5. Host cell comprising a DNA-molecule of claim 1, a multicopy vector of claim 3 and/or a plasmid of claim 4, as a part of its chromosome, as a part of a mini-chromosome, or extra-chromosomally.

6. Host cell according to claim 5, wherein said host cell is a yeast.

7. Host cell according to claim 6 selected from the group comprising of S. cerevisiae, K. lactis, H. polymorpha and P. pastoris.

8. Process of fermenting a fusion protein according to claim 2, in which
(a) a DNA-molecule of claim 1, a multicopy vector of claim 3, or a plasmid of claim 4 is expressed in a host cell according to any of claims 5 to 7, and
(b) the expressed fusion protein is isolated from the supernatant of the cell culture.

9. Process according to claim 8, wherein after completion of fermentation, the pH is adjusted to 2,5-3,5 in order to precipitate non-desired proteins and the expressed fusion protein is isolated from the supernatant of the precipitation.

10. Process according to claim 9, in which process after separating the fermentation supernatant from the host cells, the host cells are repeatedly cultured in fresh medium, and the released fusion protein is isolated from each supernatant obtained during cultivation.

11. A process according to any of the claims 8 to 10, wherein a process step for concentrating the expressed protein in the supernatant after precipitation is selected from a group comprising microfiltration, hydrophobic interaction chromatography and ion exchange chromatography.

12. Process for preparing insulin, in which
(a) a fusion protein is expressed and isolated according to claims 9 to 11;
(b) the fusion protein is treated with trypsin and carboxypeptidase B; and
(c) insulin is isolated from the reaction mixture of step (b).

## Patentansprüche

1. DNA-Molekül der Form:
Pₓ-Sₓ-Bₙ- (ZR) -Hir (AsₘR) -protein (Y) -T
worin
Pₓ für eine beliebige Promotor-DNA-Sequenz steht und so ausgewählt ist, daß optimale Ausbeuten des interessierenden Proteins erzielbar werden;
Sₓ für eine beliebige, für eine optimale Ausbeuten gestattende Signalsequenz oder Leitsequenz codierende DNA steht;
Bₙ für 1-15 Aminosäurecodons oder eine chemische Bindung steht;
Z für das Codon einer Aminosäure, ausgewählt aus der Gruppe Lys und Arg, steht;
R für ein Arg-Codon oder eine chemische Bindung steht;
Asₘ für eine chemische Bindung oder m Aminosäurecodons, mit m = 1-10, steht;
Hir für eine DNA-Sequenz steht, die für Hirudin oder ein mindestens 40% Homologie zu natürlichem Hirudin aufweisendes Hirudinderivat codiert, wobei sich das Hirudinderivat mit guten Ausbeuten, die denen für Hirudin selbst ähneln, aus Hefe exportieren läßt;
protein Y für eine ein Mini-Proinsulin codierende DNA-Sequenz steht und
T für eine nichttranslatierte DNA-Sequenz mit einem stabilisierenden Effekt auf die mRNA steht.

2. Fusionsprotein, codiert von einem der DNA-Moleküle nach Anspruch 1.

3. "Multicopy"-Vektor, umfassend das DNA-Molekül aus Anspruch 1.

4. Plasmid, umfassend das DNA-Molekül aus Anspruch 1.

5. Wirtszelle, umfassend ein DNA-Molekül aus Anspruch 1, einen Multicopy-Vektor aus Anspruch 3 und/oder ein Plasmid aus Anspruch 4 als Teil ihres Chromosoms, als Teil eines Minichromosoms oder in extrachromosomaler Form.

6. Wirtszelle nach Anspruch 5, wobei es sich um eine Hefe handelt.

7. Wirtszelle nach Anspruch 6, ausgewählt aus der Gruppe S. cerevisiae, K. lactis, H. polymorpha und P. pastoris.

8. Verfahren zur Fermentation eines Fusionsproteins nach Anspruch 2, bei dem man
a. ein DNA-Molekül aus Anspruch 1, einen Multicopy-Vektor aus Anspruch 3 oder ein Plasmid aus Anspruch 4 in einer Wirtszelle nach einem der Ansprüche 5 bis 7 exprimiert und
b. das exprimierte Fusionsprotein aus dem Überstand der Zellkultur isoliert.

9. Verfahren nach Anspruch 8, wobei nach Beendigung der Fermentation der pH-Wert zur Ausfällung unerwünschter Proteine auf 2,5-3,5 eingestellt wird und das exprimierte Fusionsprotein aus dem Überstand des Niederschlags isoliert wird.

10. Verfahren nach Anspruch 9, bei dem man nach Trennung des Fermentationsüberstands von den Wirtszellen diese wiederholt in frischem Medium kultiviert und jeweils das freigesetzte Fusionsprotein aus den während der Kultivierung erhaltenen einzelnen Überständen isoliert.

11. Verfahren nach einem der Ansprüche 8 bis 10, wobei ein Verfahrensschritt zur Anreicherung des exprimierten Proteins im Überstand nach der Ausfällung ausgewählt ist aus der Gruppe Mikrofiltration, hydrophobe Wechselwirkungschromatographie und Ionenaustauschchromatographie.

12. Verfahren zur Herstellung von Insulin, bei dem man
a. ein Fusionsprotein gemäß den Ansprüchen 9 bis 11 exprimiert und isoliert,
b. das Fusionsprotein mit Trypsin und Carboxypeptidase B behandelt und
c. Insulin aus dem Reaktionsgemisch aus Schritt (b) isoliert.

## Revendications

1. Molécule d'ADN de la forme :
Pₓ-Sₓ-Bₙ- (ZR) -Hir (AsₘR) -protéine (Y) -T
dans laquelle
Pₓ est une séquence quelconque d'ADN promoteur, choisie de telle manière que des rendements optimums de la protéine présentant un intérêt deviennent réalisables ;
Sₓ est un ADN quelconque codant pour une séquence signal ou pour une séquence de tête, qui permet d'obtenir des rendements optimums ;
Bₙ est (sont) de 1 à 15 codon(s) codant pour des acides aminés ou une liaison chimique ;
Z est le codon d'un acide aminé choisi dans le groupe comprenant Lys et Arg ;
R est un codon d'Arg ou une liaison chimique ;
Asₘ est une liaison chimique ou m codons codant pour des acides aminés, où m = 1 à 10 ;
Hir est une séquence d'ADN codant pour l'hirudine ou un dérivé de l'hirudine qui est homologue, au moins à 40%, à l'hirudine naturelle, le dérivé de l'hirudine pouvant être obtenu de la levure avec de bons rendements, semblables à ceux de l'hirudine elle-même ;
la protéine Y est une séquence d'ADN codant pour une mini-proinsuline ; et
T est une séquence d'ADN non traduite ayant un effet stabilisateur sur l'ARNm.

2. Protéine de fusion codée par n'importe laquelle des molécules d'ADN selon la revendication 1.

3. Vecteur multicopie comprenant la molécule d'ADN selon la revendication 1.

4. Plasmide comprenant la molécule d'ADN selon la revendication 1.

5. Cellule hôte comprenant une molécule d'ADN, selon la revendication 1, un vecteur multicopie, selon la revendication 3, et/ou un plasmide, selon la revendication 4, comme partie de son chromosome, comme partie d'un mini-chromosome ou de manière extra-chromosomique.

6. Cellule hôte selon la revendication 5, dans laquelle ladite cellule hôte est une levure.

7. Cellule hôte, selon la revendication 6, choisie dans le groupe comprenant *S*. *cerevisiae, K. lactis, H*. *polymorpha* et *P*. *pastoris.*

8. Processus de fermentation d'une protéine de fusion selon la revendication 2, dans lequel :
(a) une molécule d'ADN, selon la revendication 1, un vecteur multicopie, selon la revendication 3, ou un plasmide, selon la revendication 4, est exprimé(e) dans une cellule hôte, selon l'une quelconque des revendications 5 à 7, et
(b) la protéine de fusion exprimée est isolée à partir du surnageant de la culture de cellules.

9. Processus selon la revendication 8, dans lequel, après la fin de la fermentation, le pH est ajusté jusqu'à 2,5-3,5 dans le but de précipiter les protéines non souhaitées ; et la protéine de fusion exprimée est isolée à partir du surnageant de précipitation.

10. Processus selon la revendication 9, dans lequel, après avoir séparé le surnageant de fermentation des cellules hôtes, les cellules hôtes sont cultivées de manière répétée dans du milieu frais et la protéine de fusion libérée est isolée à partir de chaque surnageant obtenu lors de la culture.

11. Processus selon l'une quelconque des revendications 8 à 10, dans lequel une étape du processus pour concentrer la protéine exprimée dans le surnageant après précipitation est choisie dans un groupe comprenant : la microfiltration ; une chromatographie à interactions hydrophobes ; et une chromatographie échangeuse d'ions.

12. Processus pour préparer de l'insuline, dans lequel :
(a) une protéine de fusion est exprimée et isolée selon les revendications 9 à 11 ;
(b) la protéine de fusion est traitée avec de la trypsine et une carboxypeptidase B ; et
(c) l'insuline est isolée à partir du mélange de réaction de l'étape (b).


### SEQUENCE LISTING

<110> Aventis Pharma Deutschland GmbH
<120> Use of fusion proteins whose N-terminal part is a hirudin derivative for the production of recombinant proteins via secretion by yeasts
<130> DEAV2001/0008
<140> 10108211.8
   <141> 2001-02-20
<160> 11
<170> PatentIn Ver. 2.1
<210> 1
   <211> 47
   <212> DNA
   <213> Artificial Sequence
<220>
   <223> Description of Artificial Sequence:hir_insf1
<400> 1
   atccctgagg aataccttca gcgatttgtt aaccaacact tgtgtgg 47
<210> 2
   <211> 15
   <212> PRT
   <213> Artificial Sequence
<220>
   <223> Description of Artificial Sequence:protein hir_insf1
<400> 2
   Ile Pro Glu Glu Tyr Leu Gln Arg Phe Val Asn Gln His Leu Cys
   1 5 10 15
<210> 3
   <211> 47
   <212> DNA
   <213> Artificial Sequence
<220>
   <223> Description of Artificial Sequence: hir_insrev1
<400> 3
   cctcacaagt gttggttaac aaatcgctga aggtattcct cagggat 47
<210> 4
   <211> 46
   <212> DNA
   <213> Artificial Sequence
<220>
   <223> Description of Artificial Sequence: hirf1
<400> 4
   tttttttgga tcctttggat aaaagactta cgtatactga ctgcac 46
<210> 5
   <211> 6
   <212> PRT
   <213> Artificial Sequence
<220>
   <223> Description of Artificial Sequence: protein hirf1
<400> 5
   Leu Thr Tyr Thr Asp Cys
   1 5
<210> 6
   <211> 24
   <212> DNA
   <213> Artificial Sequence
<220>
   <223> Description of Artificial Sequence: insncolrev
<400> 6
   ttttttccat gggtcgacta tcag 24
<210> 7
   <211> 59
   <212> DNA
   <213> Artificial Sequence
<220>
   <223> Description of Artificial Sequence: Hir_insf
<400> 7
   atccctgagg aataccttca gggaaattcg gcacgatttg ttaaccaaca cttgtgtgg 59
<210> 8
   <211> 12
   <212> PRT
   <213> Artificial Sequence
<220>
   <223> Description of Artificial Sequence: protein Hir_insf
<400> 8
   Gly Asn Ser Ala Arg Phe Val Asn Gln His Leu Cys
   1 5 10
<210> 9
   <211> 59
   <212> DNA
   <213> Artificial Sequence
<220>
   <223> Description of Artificial Sequence: Hir_insrev
<400> 9
   ccacacaagt gttggttaac aaatcgtgcc gaatttccct gaaggtattc ctcagggat 59
<210> 10
   <211> 34
   <212> DNA
   <213> Artificial Sequence
<220>
   <223> Description of Artificial Sequence: pichia_H_lf1
<400> 10
   tttttttctc gagaaaagac ttacgtatac tgac 34
<210> 11
   <211> 41
   <212> DNA
   <213> Artificial Sequence
<220>
   <223> Description of Artificial Sequence: pichia_H_Irev2
<400> 11
   ttttttggcg ccgaattcac tattagttac agtagttttc c 41